(19) Europäisches Patentamt
European Patent Office
Office européen des brevets

(11) EP 0 550 279 B1

(12) EUROPEAN PATENT SPECIFICATION

(45) Date of publication and mention of the grant of the patent: **08.05.1996 Bulletin 1996/19**

(51) Int Cl.6: **C11D 1/66**, A61K 7/08

(21) Application number: **92311863.2**

(22) Date of filing: **30.12.1992**

(54) **Compositions comprising a glyceroglycolipid**

Zusammensetzungen enthaltend ein Glyceroglycolipid

Compositions contenant une glyceroglycolipid

(84) Designated Contracting States:
**CH DE ES FR GB IT LI NL SE**

(30) Priority: **31.12.1991 US 816423**
**25.11.1992 US 982199**

(43) Date of publication of application:
**07.07.1993 Bulletin 1993/27**

(73) Proprietors:
- **UNILEVER PLC**
**London EC4P 4BQ (GB)**
Designated Contracting States:
**GB**
- **UNILEVER N.V.**
**NL-3013 AL Rotterdam (NL)**
Designated Contracting States:
**CH DE ES FR IT LI NL SE**

(72) Inventors:
- **Humphreys, Robert William**
**Oradell, New Jersey 07649 (US)**
- **Wu, Shang-Ren**
**Mahwah, New Jersey 07649 (US)**
- **Hung, Anthony**
**New City, New York 10956 (US)**
- **Khan-Lodhi, Abid Nadin**
**Chester CH2 2JD (GB)**

(74) Representative: **Fransella, Mary Evelyn et al**
**Unilever PLC**
**Patent Division**
**Colworth House**
**Sharnbrook**
**Bedford MK44 1LQ (GB)**

(56) References cited:
**EP-A- 0 005 004**

- **DATABASE WPIL Week 9217, Derwent Publications Ltd., London, GB; AN 92-136762**
- **DATABASE WPIL Week 8419, Derwent Publications Ltd., London, GB; AN 84-116662**
- **PATENT ABSTRACTS OF JAPAN vol. 13, no. 273 (C-609)(3621) 1987**
- **PATENT ABSTRACTS OF JAPAN vol. 140, no. 30 (C-678)1989**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

The present invention relates to glyceroglycolipid compounds having an amine linkage and to detergent or personal product compositions comprising these glyceroglycolipids as surfactants or cosurfactants in the compositions.

Natural glycolipids are known in the art and these structures have been elucidated. The term glycolipid refers to any of a class of lipids that, upon hydrolysis, yield a sugar (eg galactose or glucose), and a lipid (eg substituted glycerol group). One major class of these glycolipids belong to the glycero glycolipids, ie a glycolipid based around a glycerol frame structure. For example, the compound may have a sugar structure at one end of the glycerol structure instead of an -OH group and an ester linkage at one or both of the other -0H groups that would normally be found on glycerol.

US 3 729 461 (Pomeranz et al), for example, teaches mono- and di-galactosyl glyceride compounds isolated from wheat flour. On one end of the glycerol frame is found a sugar group (ie the mono- or di-saccharide group) and the two other OH groups normally found on a glycerol are esterified.

In Kobayashi et al., J. Chem Soc. Perkin., Trans. p. 101-103 (1989), there are again taught mono- and di-galactosyl diacylglycerols similar to chose taught in Pomeranz et al. Again, there is a sugar group on one end and a mono- or diester where the remaining two -0H groups on a glycerol would normally be found.

Other ester functionalized mono- and diacyl galactosylglycerols are taught in Baruah et al., Phytochemistry, 22(8): 1741-1744 (1983) and in U.S. Patent No. 4,859,589 to Godfretsen et al.

Another example of a glyceroglycolipid is a compound having a sugar structure at one end of a glycerol structure instead of an -OH group and ether linkage on one or both of the other -OH groups that would normally be found on glycerol.

A glyceroglycolipid containing an ether linkage (where the -0H group on the glycerol would normally be found) is disclosed in Coulon-Moulec, Bull. Soc. Chem. Biol., 49(7):825-840 (1967); and in Alvarez et al., J. Lipid Res., 31(6): 1073-1081 (1990).

These references are concerned, however, only with the synthesis of various lipid glycosides and contains absolutely no teaching or suggestion that glyceroglycolipids having an ether linkage can be used as surfactants or cosurfactants in detergent or personal product compositions.

EP 232 851A (National Starch) also appears to teach a glyceroglycolipid with an ether linkage. However, this reference is clearly concerned with compounds used as paper strength additives and neither teaches nor suggests that these compounds may be used as surfactants in detergent or personal wash compositions.

Other examples of glyceroglycolipids having an ether linkage are found in our copending application (Case C6175) filed on the same date as the present application.

William et al., Archives of Biochemistry and Biophysics, 195(1):145-151 (1979) teach certain alkyl bionamide compounds which are formed by linking aldobionic acids to an alkylamine through an amide bond. The compounds with which the present invention is concerned contain no such amide bond.

Scardera et al (US 5 023 008) teach a glycerol like frame structure with an amine group on one end. However, this structure has no sugar group on the other end.

Yet another example of a glyceroglycolipid might be a compound with a sugar group on one end of the glycerol structure instead of an -OH group and an amine group (ie $NRR_1$) at one or both of the other -OH groups normally found in glycerol, and in particular amines wherein one or both of R and $R_1$ are alkyl chains sufficient in length to confer surfactancy properties on the molecule.

US 4 011 169 (Diehl et al/Procter & Gamble) teaches enzyme containing compositions comprising certain aminated polysaccharides as stabilising agents for enzymes. It is clear from this reference that the polysaccharides used have at the very least 5 or more saccharide units and, preferably, well over 100 (the application notes at column 7, lines 50-52, that natural polysaccharides smaller than this are rare). Further, there is a limitation to the amount of elemental nitrogen in the compound and it seems that compounds with fewer saccharide units would not meet this limitation.

US 3 931 148 (Langdon/BASF Wyandotte) teaches hydroxyalkylamino glycoside compounds which appear to be similar to those of the invention. However, there appears to be no teaching or suggestion that these compounds can be used in the specifically claimed detergent and personal product compositions of the invention.

Finally, because these compounds are derived from naturally occurring carbohydrates, the use of these compounds can provide a source of renewable raw materials that are synthetically versatile and environmentally friendly.

### DEFINITION OF THE INVENTION

The present invention provides a detergent or cleansing composition having a surfactant system comprising a glyceroglycolipid surfactant having an amine linkage, having the formula given below, together with a cosurfactant selected from fatty acid soaps, anionic non-soap surfactants, nonionic surfactants, cationic surfactants, amphoteric

surfactants and zwitterionic surfactants.

The glyceroglycolipid surfactant has the structure:

$$\begin{array}{c} \quad\quad\quad\ \ B \\ \quad\quad\quad\ \ | \\ A^1\text{-O-}CH_2\text{-}CH\text{-}CH_2NRR_1 \end{array}$$

wherein $A^1$ is a saccharide, R or $R_1$ are the same or different and are hydrogen or a branched or unbranched, saturated or unsaturated, hydrocarbon radical having from 1 to 24 carbon atoms; and B is OH or $NR_2R_3$, wherein $R_2$ and $R_3$ are the same or different and are hydrogen, a branched or unbranched hydrocarbon, saturated or unsaturated radical having from 1 to 24 carbons; wherein the $NRR_1$ group and B group are positionally interchangeable; and wherein at least one of R, $R_1$, $R_2$ or $R_3$ must be a hydrocarbon radical having from 1 to 24 carbon atoms.

## DETAILED DESCRIPTION OF THE INVENTION

The glyceroglycolipid compounds used as surfactants in accordance with the present invention have one or two amine linkages. In the formula given above, the radicals R and $R_1$ preferably have from 6 to 18 carbon atoms and can be aryl, aralkyl etc; and when B is a $NR_2R_3$ group $R_2$ and $R_3$ preferably have from 6 to 18 carbons. It should be understood that the $NRR_1$ group and the B group are positionally interchangeable.

It should also be understood that at least one of R, $R_1$, $R_2$ or $R_3$ must be a hydrocarbon radical (ie they may not all be hydrogen at the same time) in order to ensure surfactancy behaviour. Preferably, at least one of these groups is $C_6$-$C_{22}$, most preferably $C_{10}$ - $C_{14}$.

Preferably the compound is a monoalkylamine such that R (or $R_1$) is hydrogen and $R_1$ (or R) is an alkyl chain having, for example, 10 to 14 carbons.

In a preferred embodiment of the invention, $A^1$ is a monosaccharide and, in particular, is a galactoside (eg D-galactoside), R is a $C_{12}$ alkyl chain, $R_1$ is hydrogen and B is OH.

Other examples of compounds of the invention (having varying $NRR_1$ or $A^1$groups) are set forth below:

3-(butylamino) -2-hydroxypropyl-beta-D-galactopyranoside

3-(pentylamino)-2-hydroxypropyl-beta-D-galactopyranoside

3-(hexylamino)-2-hydroxypropyl-beta-D-galactopyranoside

3-(heptylamino)-2-hydroxypropyl-beta-D-galactopyranoside

3-(octylamino)-2-hydroxypropyl-beta-D-galactopyranoside

3-(nonylamino)-2-hydroxypropyl-beta-D-galactopyranoside

3-(decylamino)-2-hydroxypropyl-beta-D-galactopyranoside

3-(dodecylamino)-2-hydroxypropyl-beta-D-galactopyranoside

3-(tetradecylamino)-2-hydroxypropyl-beta-D-galactopyranoside

3-(hexadecylamino)-2-hydroxypropyl-beta-D-galactopyranoside

3-(octadecylamino)-2-hydroxypropyl-beta-D-galactopyranoside

3-(eicosylamino)-2-hydroxypropyl-beta-D-galactopyranoside

3-(docosylamino)-2-hydroxypropyl-beta-D-galactopyranoside

3-(tetracosylamino)-2-hydroxypropyl-beta-D-galactopyranoside

3-(hexenylamino)-2-hydroxypropyl-beta-D-galactopyranoside

3-(decenylamino)-2-hydroxypropyl-beta-D-galactopyranoside

3-(dodecenylamino)-2-hydroxypropyl-beta-D-galactopyranoside

3-(tetradecenylamino)-2-hydroxypropyl-beta-D-galactopyranoside

3-(hexadecenylamino)-2-hydroxypropyl-beta-D-galactopyranoside

3-(octadecenylamino)-2-hydroxypropyl-beta-D-galactopyranoside

3-(docosenylamino)-2-hydroxypropyl-beta-D-galactopyranoside

3-(tetracosenylamino)-2-hydroxypropyl-beta-D-galactopyranoside

3-(3-oxa-tridecylamino)-2-hydroxypropyl-beta-D-galactopyranoside

3-(fluorododecylamino)-2-hydroxypropyl-beta-D-galactopyranoside

3-(butylamino)-2-hydroxypropyl-beta-D-glucopyranoside

3-(pentylamino)-2-hydroxypropyl-beta-D-mannopyranoside

SYNTHESIS OF THE GLYCEROGLYCOLIPID SURFACTANT

The glyceroglycolipid with which the invention is concerned may be formed from a precursor having an epoxide group at the location where the amine linkage is formed and having a sugar group. The sugar may be protected or unprotected. An example of such a precursor would be the beta-D-galactose epoxide compound having the structure:

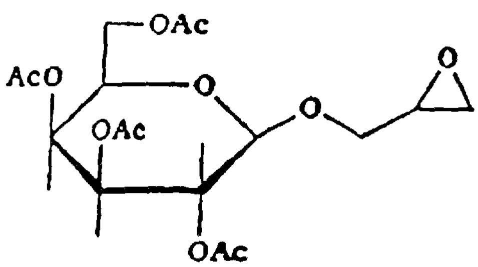

Once the protected epoxide galactose compound is obtained, this can be reacted with a primary or secondary amine $NH_2R$ or $NHRR_1$ (wherein R and $R_1$ represents the desired chain length of the alkyl groups forming the amine linkage). The amine is added in an inert solvent (e.g. tetrahydrofuran, acetonitrile, dioxane) or neat.

The epoxide precursor used to form the desired surfactant can in turn be formed in a variety of ways.

For example, a galactose epoxide compound may be synthesized enzymatically via the hydrolysis of lactose in the presence of allyl alcohol and beta-galactosidase to form a allyl-beta-D-galactopyranoside which can then be protected and oxidized to the corresponding epoxide with m-chloroperoxybenzoic acid (m-CPBA) in dichloromethane.

This type of reaction, which is taught in Nilsson, K.G.I., Carbohydrate Research, 180:53-59 (1988) is set forth below:

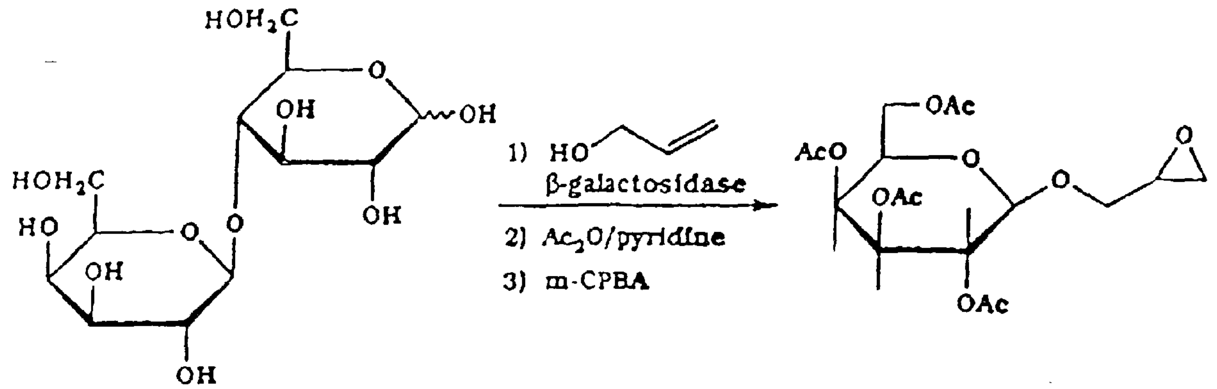


A chemical mode for preparation of the galactose epoxide involves the use of acetobromogalactose (2,3,4,6-tetra-O-acetyl-a-D-galactopyranosyl bromide) mixed with allyl alcohol and mercuric cyanide. This simple Koenigs-Knorr glycosydation affords the allyl-beta-D-galactopyranoside tetraacetate in very good yield. Oxidation with peracide gives the protected epoxide sugar.

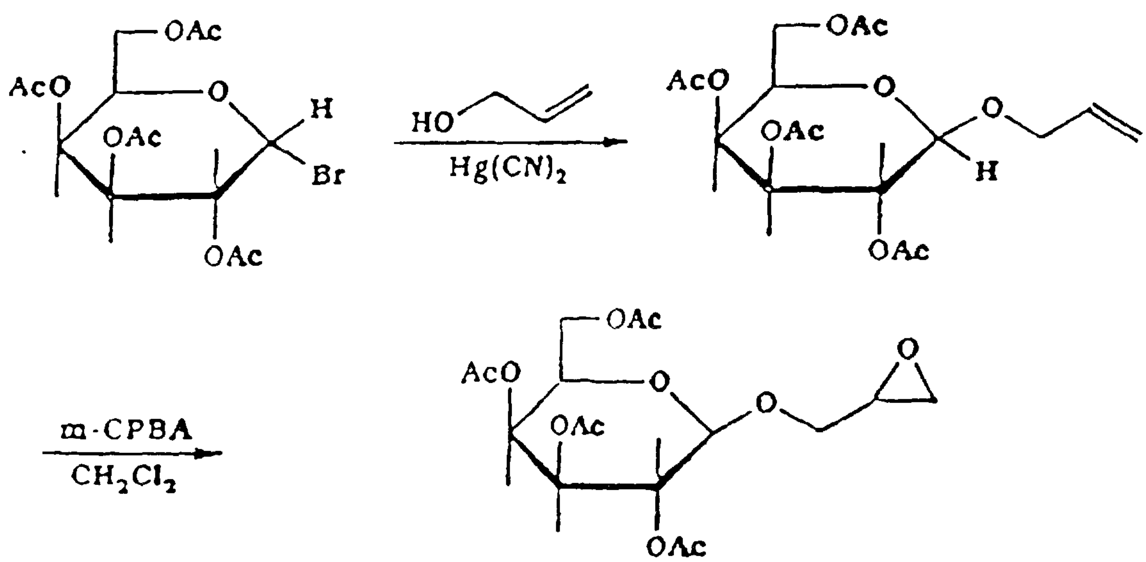


Once the epoxide precursor is formed, the epoxide is opened with an amine with formation of the amine bond and one free hydroxy group.

DETERGENT COMPOSITIONS

The glyceroglycolipid surfactants with cosurfactants may be used in cleansing or detergent compositions for fabrics or for hard surfaces.

Examples of liquid detergent compositions are described in US 4 959 179 (Aronson et al). hereby incorporated by reference into the subject application; and examples of powdered detergent compositions are described in US 4 929 379 (Oldenburg et al), hereby incorporated by reference into the subject application.

The liquid detergent compositions of the invention may be built or unbuilt and may be aqueous or nonaqueous. The compositions generally comprise about 5-70% by weight of a detergent active material and from 0 to 50 wt% of a builder. The liquid detergent compositions of the invention may further comprise an amount of electrolyte (defined as any water-soluble salt) whose quantity depends on whether or not the composition is structured. By structured is meant the formation of a lamellar phase sufficient to endow solid suspending capability.

More particularly, while no electrolyte is required for a non-structured, non-suspending composition, at least 1%, more preferably at least 5% by weight and most preferably at least 15% by weight electrolyte is used. The formation of a lamellar phase can be detected by means well known to those skilled in the art.

The water-soluble electrolyte salt may be a detergency builder, such as the inorganic salt sodium tripolyphosphate or it may be a non-functional electrolyte such as sodium sulphate or chloride. Preferably, whatever builder is used in the composition comprises all or part of the electrolyte.

The liquid detergent composition generally further comprises enzymes such as proteases, lipases, amylases and cellulases which, when present, may be used in amounts from 0.01 to 5% of the compositions. Stabilisers or stabiliser systems may be used in conjunction with enzymes and generally comprise from 0.1 to 15% by weight of the composition.

The enzyme stabilisation system may comprise calcium ion, boric acid, propylene glycol and/or short chain carboxylic acids. The composition preferably contains from 0.01 to 50, preferably from 0.1 to 30, more preferably from 1 to 20 millimoles of calcium ion per litre.

When calcium ion is used, the level of calcium ion should be selected so that there is always some minimum level available for the enzyme after allowing for complexation with builders, etc., in the composition. Any water-soluble calcium salt can be used as the source of calcium ion, including calcium chloride, calcium formate, calcium acetate and calcium propionate. A small amount of calcium ion, generally from 0.05 to 2.5 millimoles per litre, is often also present in the composition due to calcium in the enzyme slurry and formula water.

Another enzyme stabiliser which may be used is propionic acid or a propionic acid salt capable of forming propionic acid. When used, this stabiliser may be used in an amount from 0.1 to 15% by weight of the composition.

Other preferred enzyme stabilisers are polyols containing only carbon, hydrogen and oxygen atoms. They preferably contain from 2 to 6 carbon atoms and from 2 to 6 hydroxy groups. Examples include propylene glycol (especially 1,2 propanediol which is preferred), ethylene glycol, glycerol, sorbitol, mannitol and glucose. The polyol generally represents from 0.5% to 15%, preferably from 1.0 to 8% by weight of the composition.

The composition herein may also optionally contain from 0.25 to 5 wt%, most preferably from 0.5 to 3% by weight of boric acid. The boric acid may be, but is preferably not, formed by a compound capable of forming boric acid in the composition. Boric acid is preferred, although other compounds such as boric oxide, borax and other alkali metal borates (e.g. sodium ortho-, meta- and pyroborate and sodium pentaborate) are suitable. Substituted boric acids (for example, phenylboronic acid, butane boronic acid and a p-bromo phenylboronic acid) can also be used in place of boric acid.

On especially preferred stabilisation system is a polyol in combination with boric acid. Preferably, the weight ratio of polyol to boric acid added is at least 1, more preferably at least about 1.3.

With regard to the detergent active system, the cosurfactant may be an alkali metal or alkanolamine soap or a 10 to 24 carbon atom fatty acid, including polymerised fatty acids, or an anionic, a nonionic, cationic, zwitterionic or amphoteric synthetic detergent material, or mixtures of any of these.

Examples of the anionic synthetic detergents are salts (including sodium, potassium, ammonium and substituted ammonium salts) such as mono-, di- and triethanolamine salts of 9 to 20 carbon alkylbenzenesulphonates, 8 to 22 carbon primary or secondary alkanesulphonates, 8 to 24 carbon olefinsulphonates, sulphonated polycarboxylic acids prepared by sulphonation of the pyrolyzed product of alkaline earth metal citrates, for example, as described in GB 1 082 179, 8 to 22 carbon alkylsulphates, 8 to 24 carbon alkylpolyglycol-ether-sulphates, -carboxylates and - phosphates (containing up to 10 moles of ethylene oxide); further examples are described in "Surface Active Agents and Detergents" (vol. I and II) by Schwartz, Perry and Berch. Any suitable anionic may be used and the examples are not intended to be limiting in any way.

Examples of nonionic synthetic detergents which may be used with the invention are the condensation products of ethylene oxide, propylene oxide and/or butylene oxide with 8 to 18 carbon alkylphenols, 8 to 18 carbon fatty acid amides; further examples of nonionics include tertiary amine oxides with 8 to 18 carbon alkyl chain and two 1 to 3 carbon alkyl chains. The above reference also describes further examples of nonionics. Preferred nonionic surfactants are ethoxylated alcohols, for example, $C_{12}$ alcohol 3EO ethoxylate.

The average number of moles of ethylene oxide and/or propylene oxide present in the above nonionics varies from 1-30; mixtures of various nonionics, including mixtures of nonionics with a lower and a higher degree of alkoxylation, may also be used.

Examples of cationic detergents which may be used are the quaternary ammonium compounds such as alkyldimethylammonium halogenides.

Examples of amphoteric or zwitterionic detergents which may be used with the invention are N-alkylamine acids, sulphobetaines, condensation products of fatty acids with protein hydrolysates; but owing to their relatively high costs they are usually used in combination with an anionic or a nonionic detergent. Mixtures of the various types of active detergents may also be used, and preference is given to mixtures of an anionic and a nonionic detergent active. Soaps (in the form of their sodium, potassium and substituted ammonium salts) of fatty acids may also be used, preferably in conjunction with an anionic and/or nonionic synthetic detergent.

Builders which can be used according to this invention include conventional alkaline detergency builders, inorganic or organic, which can be used at levels from 0 to 50% by weight of the composition, preferably from 1 to 20% by weight, most preferably from 2 to 8%.

Examples of suitable inorganic alkaline detergency builders are water-soluble alkalimetal phosphates, polyphosphate, borates, silicates and also carbonates. Specific examples of such salts are sodium and potassium triphosphates, pyrophosphates, orthophosphates, hexametaphosphates, tetraborates, silicates and carbonates.

Examples of suitable organic alkaline detergency builder salts are: (1) water-soluble amino polycarboxylates, for example, sodium and potassium ethylenediaminetetraacetates, nitrilotriacetates and N-(2 hydroxyethyl)-nitrilodiacetates; (2) water-soluble salts of phytic acid, for example, sodium and potassium phytates (see US 2 379 942); (3) water-soluble polyphosphonates, including specifically, sodium, potassium and lithium salts of ethane-1-hydroxy-1,1diphosphonic acid; sodium, potassium and lithium salts of methylene diphosphonic acid; and sodium, potassium and lithium salts of ethane-1,1,2-triphosphonic acid. Other examples include the alkali methal salts of ethane-2-carboxy-1,1-diphosphonic acid hydroxymethanediphosphonic acid, carboxylidiphosphonic acid, ethane-1-hydroxy-1,1,2-triphosphonic acid, ethane-2-hydroxy-1,1,2-triphosphonic acid, propane-1,1,3,3-tetraphosphonic acid, propane-1,1,2,3-tetraphosphonic acid, and propane-1,2,2,3-tetraphosphonic acid; (4) water soluble salts of polycarboxylate polymers and copolymers as described in US 3 308 067.

In addition, polycarboxylate builders can be used satisfactorily, including water-soluble salts of mellitic acid, citric acid, and carboxymethyloxysuccinic acid and salts of polymers of itaconic acid and maleic acid. Other polycarboxylate builders include DPA (dipicolinic acid) and ODS (oxydisoccinic acid). Certain zeolites or aluminosilicates can be used. One such aluminosilicate which is useful in the compositions of the invention is an amorphous water-insoluble hydrated compound of the formula $Na_x(Al0_2.SiO_2)_y$, wherein x is a number from 1.0 to 1.2 and y is 1, said amorphous material being further characterized by a Mg++ exchange capacity of from about 50mg eq. $CaCO_3$/g. and a particle diameter of from 0.01 micron to 5 microns. This ion exchange builder is more fully described in GB 1 470 250 (Procter & Gamble).

A second water-insoluble synthetic aluminosilicate ion exchange material useful herein is crystalline in nature and has the formula $Na_z\ [(AlO_2)_y.(SiO_2)].xH_20$, wherein z and y are integers of at least 6; the molar ratio of z and y is in the range from 1.0 to 0.5, and x is an integer from 15 to 264; said aluminosilicate ion exchange material having a particle size diameter from 0.1 micron to 100 microns; a calcium ion exchange capacity on an anhydrous basis of at least 200 milligrams equivalent of $CaCO_3$ hardness per gram; and a calcium exchange rate on an anhydrous basis of at least 2 grains/gallon/minute/gram. These synthetic aluminosilicates are more fully described in GB 1 429 143 (Procter & Gamble).

In addition to the ingredients described hereinbefore, the preferred compositions herein may frequently contain a series of optional ingredients which are used for the known functionality in conventional levels. While the detergent compositions are generally premised on aqueous, enzyme-containing detergent compositions, it is frequently desirable to use a phase regulant. This component together with water constitutes then the solvent matrix for the claimed liquid compositions. Suitable phase regulants are well-known in liquid detergent technology and, for example, can be represented by hydrotropes such as salts of alkylarylsulphonates having up to 3 carbon atoms in the alkyl group, for example, sodium, potassium, ammonium and ethanolamine salts of xylene-, toluene-, ethylbenzene-, cumene-, and isopropylbenzene sulphonic acids. Alcohols may also be used as phase regulants. This phase regulant is frequently used in an amount from 0.5 to 20 wt%, the sum of phase regulant and water normally being in the range of from 35 to 65wt%.

The preferred compositions herein can contain a series of further optional ingredients which are mostly used in additive levels, usually below 5 wt%. Examples of the like additives include: polyacids, suds regulants, opacifiers, antioxidants, bactericides, dyes, perfumes, brighteners and the like.

The beneficial utilization of the claimed compositions under various usage conditions can require the utilization of a suds regulant. While generally all detergent suds regulants can be utilized, preferred for use herein are alkylated polysiloxanes such as dimethylpolysiloxane, also frequently termed silicones. The silicones are frequently used in a level not exceeding 0.5 wt%, most preferably from 0.01 to 0.2 wt%.

It can also be desirable to utilize opacifiers inasmuch as they contribute to create a uniform appearance of the concentrated liquid detergent compositions. Examples of suitable opacifiers include: polystyrene commercially known as LYTRON (Trade Mark) 621 manufactured by Monsanto Chemical Corporation. The opacifiers are frequently used in an amount from 0.3 to 1.5 wt%.

The compositions herein can also contain known antioxidants for their known utility, frequently radical scavengers, in the art established levels, i.e., 0.001 to 0.25 wt% (by reference to total composition). These antioxidants are frequently introduced in conjunction with fatty acids.

Liquid detergent compositions of the invention may also contain deflocculating polymers such as described in US 5 071 586 (Lever Brothers Company).

When the liquid composition is an aqueous composition, the balance of the formulation consists of an aqueous medium. When it is in the form of a nonaqueous composition, the above ingredients make up for the whole formulation (a nonaqueous composition may contain up to 5 wt% water).

An ideal liquid detergent composition might contain (all percentages by weight):

(1) 5-70% detergent active system;
(2) 0-50% builder;
(3) 0-40% electrolyte
(4) 0.01-5% enzyme;
(5) 0.1-15% enzyme stabiliser;
(6) 0-20% phase regulant; and
(7) remainder water and minors

The detergent composition of the invention might also be a powdered detergent composition.

Such powdered compositions generally comprise from 5 to 40 wt% of a detergent active system which generally consists of an anionic, a nonionic active, a fatty acid soap or mixtures thereof; from 20-70% of an alkaline buffering agent; up to 60 wt% of builder, preferably 10 to 60 wt% and preferably up to 40 wt%; and balance minors and water.

The alkaline buffering agent may be any such agent capable of providing a 1% product solution with a pH of above 11.5 or even 12. Advantageous alkaline buffering agents are the alkalimetal silicates, as they decrease the corrosion of metal parts in washing machines, and in particular sodium orthometa- or di-silicates, of which sodium metasilicate is preferred. The alkaline buffering agent is suitably present in an amount of from 0 to 70% by weight, preferably from 0 to 30% by weight.

In addition the compositions of the invention can and normally will contain detergency builders in an amount of up to 60% by weight, preferably from 10 to 60 wt% and more preferably up to 40 wt% by weight of the total composition.

Suitable builders include sodium, potassium and ammonium or substituted ammonium pyro- and tri-polyphosphates, -ethylene diamine tetraacetates, -nitrilotriacetates, -etherpolycarboxylates, -citrates, -carbonates, -orthophosphates, -carboxymethyloxysuccinates, etc. Other builders include DPA and ODS. Also less soluble builders may be included, such as for example, an easily dispersible zeolite. Particularly preferred are the polyphosphate builder salts, nitrilotriacetates, citrates, carboxymethyloxysuccinates and mixtures thereof.

Other conventional materials may be present in minor amounts, provided they exhibit a good dissolving or dispersing behaviour; for example sequestering agents, such as ethylenediamine tetraphosphonic acid; soil-suspending agents, such as sodiumcarboxymethylcellulose, polyvinylpyrrolidone or the maleic anhydride/ vinylmethylether copolymer, hydrotropes; dyes; perfumes; optical brighteners; alkali-stable enzymes; germicides; anti-tarnishing agents; lather depressants; fabric softening agents; oxygen- or chlorine-liberating bleaches, such as dichlorocyanuric acid salts or alkalimetal hypochlorides.

The remainder of the composition is generally water, which may be present at least in part as bound water of hydration.

An ideal powdered detergent composition might contain the following (all percentages by weight):

(1) 5-40% detergent active system;
(2) 0-60% builder;
(3) 0-30% buffer salt;
(4) 0-30% sulphate;
(5) 0-20% bleach system;
(6) 0-4% enzyme; and
(7) minors plus water to 100%.

In another embodiment of the invention, the glycolipid surfactant may be used in a light duty liquid detergent composition such as those taught in US 4 671 894 (Lamb et al), US 4 368 146 (Aronson et al), and US 4 555 366 (Bissett et al).

Generally such compositions comprise a mixture of sulphate and sulphonate anionic surfactants together with a suds stabilising agent. These compositions may also comprise nonionic surfactants designed to reduce the level of non-performing ingredients such as solvents and hydrotropes and zwitterionic surfactants for providing enhanced grease and particulate soil removal performance.

Among other ingredients which may also be used in such compositions are opacifiers (e.g. ethylene glycol distearate), thickeners (for example, guar gum), antibacterial agents, antitarnish agents, heavy metal chelators (e.g. ETDA), perfumes and dyes.

PERSONAL PRODUCT COMPOSITIONS

Personal cleansing compositions of the invention may be, for example, toilet bar compositions, facial or body cleansing compositions, shampoos for hair or body, or dental compositions.

In one embodiment of the invention, the glyceroglycolipids with cosurfactants of the invention may be used, for

example, in a toilet bar (ie detergent and/or soap bar) formulation.

Typical toilet bar compositions are those comprising fatty acid soaps used in combination with a detergent other than fatty acid soap and free fatty acids. It should be noted that the composition may comprise no fatty acid soap and may be based on actives other than fatty acid soap. Mildness improving salts, such as alkali metal salt or isethionate, are also typically added. In addition other ingredients, such as germicides, perfumes, colorants, pigments, suds-boosting salts and anti-mushing agents may also be added.

Fatty acid soaps are typically alkali metal or alkanol ammonium salts of aliphatic alkane or alkene monocarboxylic acids. Sodium, potassium, mono-, di- and tri-ethanol ammonium cations, or combinations thereof, are suitable for purposes of the invention. The soaps are well known alkali metal salts of natural or synthetic aliphatic (alkanoic or alkenoic) acids having 8 to 22 carbon atoms, preferably 12 to 18 carbon atoms.

Examples of soap which may be used may be found in US 4 695 395 (Caswell et al) and US 4 260 507 (Barrett).

In a soap-based bar, fatty acid soaps will generally comprise greater than 25 wt% of the composition, generally from 30-95 wt%. Preferably, the amount of soap will range from 40 to 70 wt% by weight of the composition.

In a bar based on other actives, soap may comprise 0-50% by weight. In general $C_{8-24}$ fatty acid comprises 5-60 wt% of the composition.

The compositions will also generally comprise a non-soap detergent which is generally chosen from anionic, nonionic, cationic, zwitterionic or amphoteric synthetic detergent materials or mixtures thereof. These surfactants are all well known in the art and are described, for example, in US 4 695 395 and US 4 260 507 discussed above. One preferred non-soap anionic is a $C_{8-22}$ alkyl isethionate. These esters may be prepared by the reaction between alkali metal isethionate and mixed aliphatic fatty acids having from 8 to 22 carbons. The non-soap actives may comprise from 0 to 50 wt% of the composition.

A certain amount of free fatty acids of 8 to 22 carbon atoms are also desirably incorporated into soap compositions to act as superfatting agents or as skin feel and creaminess enhancers. If present, the free fatty acids comprise between 1 and 15 wt% of the compositions.

A preferred mildness improving salt which may be added to soap compositions is a simple unsubstituted sodium isethionate. This may be present as 0.1 to 50 wt% of the composition, preferably 0.5 to 25 wt%, more preferably 2 to 15 wt%. Other mildness coactives which may be used include betain compounds or ether sulphates. These also may be present at 0.1 to 50 wt% of the composition, preferably 0.5 to 25 wt%.

The sulphate ester surfactant may comprise 0.01 to 45 wt% by weight of the composition (as the monoester), preferably 25 to 40 wt, and 0.01 to 10 wt% of the composition (as the diester), preferably 0.01 to 5 wt%.

Other optional ingredients which may be present in soap bar compositions are moisturisers such as glycerin, propylene glycol, sorbitol, polyethylene glycol, ethoxylated or methoxylated ether of methyl glucose etc.; water-soluble polymers such as collagens, modified cellulases (such as Polymer JR (Trade Mark)), guar gums and polyacrylates; sequestering agents such as citrate, and emollients such as silicones or mineral oil. Another useful set of ingredients are various cosurfactants and non-soap detergents.

In another embodiment of the invention, the surfactant of the invention may be present in a facial or body cleansing composition. Examples of such cleaning compositions are described, for example, in US 4 812 253 (Small et al) and US 4 526 710 (Fujisawa).

Typically, cleansing compositions will comprise a fatty acid soap together with a non-soap surfactant, preferably a mild synthetic surfactant. Cleaning compositions will also generally include a moisturiser or emollient and polymeric skin feel and mildness aids. The compositions may further optionally include thickener (eg magnesium aluminum silicate, Carbopol (Trade Mark), water soluble polymers (e.g., carboxymethylcellulose), dyes, hydrotropes, brighteners, perfumes and germicides.

The fatty acid soaps used are such as those described above in uses in detergent bar formulations. These soaps are typically alkali metal or alkanol ammonium salts of aliphatic or alkene monocarboxylic salts. Sodium, potassium, mono-, di- and triethanol ammonium cations, or combinations thereof are suitable. Preferred soaps are 8 to 24 carbon half acid salts of, for example, triethanolamine.

Surfactants can be chosen from anionic, nonionic, cationic, zwitterionic or amphoteric materials or mixtures thereof such as are described in US 4 695 395 mentioned above, or in US 4 854 333 (Inman et al).

Moisturisers are included to provide skin conditioning benefits and improve mildness. This term is often used as synonymous with emollient and is then used to describe a material which imparts a smooth and soft feeling to skin surface.

There are two ways of reducing water loss from the stratum corneum. One is to deposit on the surface of the skin an occlusive layer which reduces the rate of evaporation. The second method is to add nonocclusive hydgroscopic substances to the stratum corneum which will retain water, and make this water available to the stratum corneum to alter its physical properties and produce a cosmetically desirable effect. Nonocclusive moisturisers also function by improving the lubricity of the skin.

Both occlusive and nonocclusive moisturisers can work in the present invention. Some examples of moisturisers

are long chain fatty acids, liquid water-soluble polyols, glycerin, propylene glycol, sorbitol, polyethylene glycol, ethoxylated/propoxylated ethers of methyl glucose (eg., methyl gluceth-20) and ethoxylated/-propoxylated ethers of lanolin alcohol (eg Solulan-75).

Preferred moisturisers are coco and tallow fatty acids. Some other preferred moisturisers are the nonocclusive liquid water soluble polyols and the essential amino acid compounds found naturally in the skin.

Other preferred nonocclusive moisturisers are compounds found to be naturally occurring in the stratum corneum of the skin, such as sodium pyrrolidone carboxylic acid, lactic acid, urea, L-proline, guanidine and pyrrolidone. Examples of other nonocclusive moisturisers include hexadecyl, myristyl, isodecyl or isopropyl esters of adipic, lactic, oleic, stearic, isostearic, myristic or linoleic acids, as well as many of their corresponding alcohol esters (sodium isostearoyl-2 lactylate, sodium capryl lactylate), hydrolyzed protein and other collagen-derived proteins, aloe vera gel and acetamide MEA.

Some occlusive moisturisers include petrolatum, mineral oil, beeswax, silicones, lanolin and oil-soluble lanolin derivatives, saturated and unsaturated fatty alcohols such as behenyl alcohol, squalene and squalane, and various animal and vegetable oils such as almond oil, peanut oil, wheat germ oil, linseed oil, jojoba oil, oil of apricot pits, walnuts, palm nuts, pistachio nuts, sesame seeds, rapeseed, cade oil,corn oil, peach pit oil, poppyseed oil, pine oil, castor oil, soybean oil, avocado oil, safflower oil, coconut oil, hazelnut oil, olive oil, grape seed oil and sunflower seed oil.

Other examples of both types of moisturisers are disclosed in "Emollients -- a Critical Evaluation," by J. Mausner, Cosmetics & Toiletries, May 1981.

The polymeric skin feel and mildness aids useful in the present invention are the cationic, anionic, amphoteric, and the nonionic polymers used in the cosmetic field. Reduced skin irritation benefits as measured by patch testing of cationic and nonionic types of polymers are set out in "Polymer JR for Skin Care" Bulletin, by Union Carbide, 1977. The cationics are preferred over the others because they provide better skin feel benefits.

The amount of polymeric skin feel and mildness aids found useful in the composition of the present invention is from 0.01 to 5 wt%, preferably from 0.3 to 4 wt%. In bar compositions with less than 5.5 wt% soap, the polymer is suitably used at a level of 2 to 5 wt%, preferably 3 wt% or more.

Other types of high molecular weight polymeric skin feel and skin mildness aids, such as nonionic guar gums, Merquats (Trade Mark) 100 and 550, made by Merck & Co, Inc.; Jaguar (Trade Mark) C-14-S made by Stein Hall; Mirapol (Trade Mark) A15 made by Miranol Chemical Company, Inc.; and Galactasol (Trade Mark) 811, made by Henkel, Inc.; plus others, are usable. The polymer also provides enhanced creamy lather benefits.

Nonionic polymers found to be useful include the nonionic polysaccharides, eg nonionic hydroxypropyl guar gums, offered by Celanese Corp. A preferred nonionic hydroxypropyl guar gum material is Jaguar (Trade Mark) HP-60 having molar substitution of about 0.6. Another class of useful nonionics is the cellulosic nonionic polymers, for example, HEC and CMC.

The cationic polymers employed in this invention also provide a desirable silky, soft, smooth in-use feeling. The preferred level for this invention is 0.1-5 wt% of the composition. There is reason to believe that the positively charged cationic polymers can bind with negatively charges sites on the skin to provide a soft skin feel after use. Not to be bound by any theory, it is believed that the greater the charge density of the cationic polymer, the more effective it is for skin feel benefits.

Other suitable cationic polymers are copolymers of dimethylaminoethylmethacrylate and acrylamide and copolymers of dimethyldiallylammonium chloride and acrylamide in which the ratio of the cationic to neutral monomer units has been selected to give a copolymer having a cationic charge. Yet other suitable types of cationic polymers are the cationic starches, for example, Sta-Lok (Trade Mark) 300 and 400 made by Staley, Inc.

A more complete list of cationic polymers useful in the present invention is described in US 4 438 095 (Grollier/allec). Some of the more preferred cationics are listed in Col. 3, section 2; Col. 5, section 8; Col. 8, section 10; and Col. 9, lines 10-15 of the Grollier/allec patent.

In another embodiment of the invention, the glycolipid surfactant of the invention may be used, for example, in a hair or body shampoo. Examples of such compositions are described in US 4 854 333 (Inman) and US 4 526 710 (Fujisawa).

The shampoo compositions which may be used typically comprise a surfactant selected from any one of a wide variety of surfactants known in the art (such as those described in US 4 854 333). The shampoo compositions may additionally comprise a compound considered useful for treating dandruff, e.g. selenium sulphide.

The compositions all may also optionally comprise a suspending agent, for example, any of several acyl derivative materials or mixtures thereof. Among these are ethylene glycol esters of fatty acids having 16 to 22 carbons. Preferred suspending agents include ethylene glycol stearates, both mono-and distearate. Preferred alkanol amides are stearic monoethanolamide, stearic diethanolamide and stearic monoisopropanolamide. Still other long chain acyl derivatives include long chain esters of long chain fatty acids (for example, stearyl stearate, cetyl palmitate), glyceryl esters (for example, glyceryl distearate), and long chain esters of long chain alkanol amides (for example, stearamide DEA distearate, stearamide MEA stearate).

Still other suitable suspending agents are alkyl (16 to 22 carbon) dimethyl amine oxides, such as stearyl dimethyl amine oxide. If the compositions contain an amine oxide or a long chain acyl derivative as a surfactant, these components may also provide the suspending function and additional suspending agent may not be needed.

Xanthan gum is another agent used to suspend, for example, selenium sulphide which may be in the present compositions. This biosynthetic gum material is commercially available and is a heteropolysaccharide with a molecular weight of greater than 1 million. It is believed to contain D-glucose, D-mannose and D-glucuronate in the molar ratio of 2.8:2.0:2.0. The polysaccharide is partially acetylated with 4.7% acetyl. Supplemental information on these agents is found in Whistler, Roy L. (Editor), Industrial Gums -- Polysaccharides and Their Derivatives, Academic Press, New York, 1973. Kelco, a Division of Merck & Co., Inc., offers xanthan gum as Keltrol (Trade Mark).

A particularly preferred suspending system comprises a mixture of xanthan gum, present at a level of from 0.05 to 1.0 wt%, preferably from 0.2 to 0.4 wt%, of the compositions, together with magnesium aluminum silicate ($Al_2Mg_8Si_2$), present at a level of from 0.1 to 3.0 wt%, preferably from 0.5 to 2.0 wt%, of the compositions. Magnesium aluminum silicate occurs naturally in such smectite minerals as colerainite, saponite and sapphire. Refined magnesium aluminum silicates useful herein are readily available, for example as veegum, manufactured by RT Vanderbilt Company, Inc. Mixtures of suspending agents are also suitable for use in the compositions of this invention.

Other useful thickening agents are the cross-linked polyacrylates such as those manufactured by B F Goodrich and sold under the name Carbopol (Trade Mark).

Another optional component for use in the present compositions is an amide. The amide used in the present compositions can be any of the alkanolamides of fatty acids known for use in shampoos. These are generally mono- and diethanolamides of fatty acids having from 8 to 24 carbon atoms. Preferred are coconut monoethanolamide, lauric diethanolamide and mixtures thereof. The amide is suitably present at a level of from 1 to 10 wt% of the compositions.

The compositions may also contain nonionic polymer material which is used at a low level to aid in dispersing particles. The material can be any of a large variety of types including cellulosic materials such as hydroxypropyl methyl cellulose, carboxymethyl cellulose, hydroxyethyl cellulose and sodium carboxymethyl cellulose as well as mixtures of these materials. Other materials include alginates, polyacrylic acids, polyethylene glycol and starches, among many others. The nonionic polymers are discussed in detail in Industrial Gums, edited by Roy L Whistler, Academic Press Inc., 1973, and Handbook of Water-Soluble Gums and Resins, edited by Robert L Davidson, McGraw-Hill Inc., 1980.

When included, the nonionic polymer is suitably used at a level of from 0.001 to 0.1 wt%, preferably from 0.002 to 0.05 wt%, of the composition. Hydroxypropyl methyl cellulose is the preferred polymer.

Another suitable optional component useful in the present compositions is a nonvolatile silicone fluid.

The nonvolatile silicone fluid may be either a polyalkyl siloxane, a polyaryl siloxane, a polyalkylarly siloxane or a polyether siloxane copolymer and is suitably present at a level of from 0.1 to 10.0 wt%, preferably from 0.5% to 5.0 wt%. Mixtures of these fluids may also be used and are preferred in certain executions. The dispersed silicone particles should also be insoluble in the shampoo matrix. This is the meaning of "insoluble" as used herein.

The essentially nonvolatile polyalkyl siloxane fluids that may be used include, for example, polydimethyl siloxanes with viscosities ranging from about 5 to about 600 000 centistokes at 25°C. These siloxanes are available, for example, from the General Electric Company as the Viscasil series and from Dow Corning as the Dow Corning 200 series. The siloxane viscosity can be measured by means of a glass capillary viscometer as set forth in Dow Corning Corporate Test Method CTM0004, July 20, 1970. Preferably the viscosity of the these siloxanes range from about 350 centistokes to about 100 000 centistokes.

The essentially nonvolatile polyether siloxane copolymer that may be used is, for example, a polypropylene oxide modified dimethylpolysiloxane (for example, Dow Corning DC-1248), although ethylene oxide or mixtures of ethylene oxide and propylene oxide may also be used.

Suitable silicone fluids are described in US 2 826 551 (Geen), US 3 946 500 (Drakoff), US 4 364 837 (Pader) and GB 849 433 (Woolston), Silicon Compounds, distributed by Petrarch Systems Inc., 1984. This reference provides a very good listing of suitable silicone materials.

Another silicone material useful is silicone gum. Silicone gums are described by Petrarch and others including US 4 152 416 (Spitzer et al), and Noll, Chemistry and Technology of Silicones, Academic Press, New York, 1968. Useful silicone gums are also described in General Electric Silicone Rubber Product Data Sheets SE 30, SE 33, SE 54 and SE 76. "Silicone gum" materials denote high molecular weight polydiorganosiloxanes having a mass molecular weight of from about 200 000 to about 1 000 000. Specific examples include polydimethylsiloxane, (polydimethylsiloxane) (methylvinylsiloxane) copolymer, poly(dimethylsiloxane) (diphenyl) (methylvinylsiloxane) copolymer, and mixtures thereof. Mixtures of silicone fluids and silicone gums are also useful herein.

The shampoos herein can contain a variety of other nonessential optional components suitable for rendering such compositions more formulatable, or aesthetically and/or cosmetically acceptable. Such conventional optional ingredients are well-known to those skilled in the art and include, for example, preservatives, such as benzyl alcohol, methyl paraben, propyl paraben, and imidazolinidyl urea; cationic surfactants, such as cetyl trimethyl ammonium chloride, lauryl trimethyl ammonium chloride, tricetyl methyl ammonium chloride, stearyldimethyl benzyl ammonium chloride,

and di(partially hydrogenated tallow) dimethylammonium chloride; menthol; thickeners and viscosity modifiers, such as block polymers of ethylene oxide and propylene oxide such as Pluronic (Trade Mark) F88 offered by BASF Wyandotte, sodium chloride, sodium sulphate, propylene glycol, and ethyl alcohol; pH adjusting agents, such as citric acid, succinic acid, phosphoric acid, sodium hydroxide, sodium carbonate; perfumes; dyes; and sequestering agents, such as disodium ethylenediamine tetraacetate. Such agents generally are used individually at a level of from 0.01 to 10 wt%, preferably from 0.5 to 5.0 wt%, of the composition.

A typical shampoo composition might comprise (percentages by weight):

(1) 5-15% glycolipid surfactant
(2) 0-10% anionic coactive
(3) 0-10% amphoteric coactive
(4) 0-5% lauramide MEA
(5) 0-5% thickener
(6) 0-2% fragrance
(7) 0-1% preservative; and
(8) remainder water.

If formulated as conditioner shampoo, the composition may comprise:

(a) 5-60% surfactant (wholly the glycolipid surfactant of invention or comprising the surfactant of invention);

(b) 1-60% conditioner (e.g., cationic);

(c) 0-2% preservative (e.g., benzyl alcohol);

(d) 0-10% thickener (e.g., diethanalomide); and

(e) remainder water and minors.

In yet another embodiment of the invention, the surfactant may be used in a toothpaste composition such as is taught and is described in US 4 935 227 (Duckworth).

Such compositions generally comprise abrasive gels (e.g. calcium carbonate), oral therapeutic agents (for example, fluorine containing compound), coactives, flavoring agents, sweetening agents, humectants and binding or thickening gels.

Preferred toothpastes of this invention comprise 0 to 1.5 wt% by weight of anionic surfactant. In more preferred products the amount of anionic surfactant is 0 to 1% by weight with most preferred amounts being 0 to 0.75% by weight.

Toothpastes of this invention may include other surfactants, especially nonionic surfactants.

Toothpaste of the invention will also comprise the usual additional ingredients in particular humectant binder or thickening agent.

Humectants which may be used include glycerol, sorbitol syrup, polyethylene glycol, lactitol, xylitol or hydrogenated corn syrup. The total amount of humectant present will generally range from 10 to 85% by weight of the toothpaste.

Numerous binding or thickening agents have been indicated for use in toothpastes, preferred ones being sodium carboxymethylcellulose, cross-linked polyacrylates and xanthan gum. Others include natural gum binders such as gum tragacanth, gum karaya and gum arabic, Irish moss, alginates, and carrageenans. Silica thickening agents include the silica aerogels and various precipitated silicas. Mixtures of binders and thickeners may be used. The amount of binder and thickening agent included in a toothpaste is generally from 0.1 to 15% by weight.

EXAMPLES

The invention is set forth in greater detail in the example which follow below. These examples are merely to illustrate the invention and are not intended to be limiting in any way. In Example 3 reference will be made to Figures 1 and 2 of the accompanying drawings.

EXAMPLE 1

Synthesis of 3-(N,N-dibutylamino)-2-hydroxypropyl-beta-D-galactopyranoside

Acetobromogalactose (2,3,4,6-tetra-O-acetyl-alpha-D-galactopyranosyl bromide) was mixed with allyl alcohol and

mercuric cyanide via the Koenigs-Knorr glycosylation to obtain allyl-beta-D-galactopyranoside tetraacetate. This was followed by oxidation with 3-chloroperoxybenzoic acid in dichloromethane to obtain 2,3-epoxypropyl-beta-D-galactopyranoside, 2,3,4,6-O-tetracetate.

In a 35 ml two neck flask was added 0.32 g (0.8 mmoles) of the above identified epoxide compound and 1.5 ml of dry acetonitrile, followed by dropwise addition of N,N-dibutylamine (0.2g, 1.6 mmoles). The reaction was allowed to run at room temperature and was followed by thin layer chromotography using a 9:1 CHCl3:MeOH with a drop of ammonium hydroxide as eluent. $R_f$ value of the new product was found to be 0.36 (the $R_f$ of starting epoxide prior to consumption had been 0.80).

Column chromatography was used to separate the ring-opened adduct from the excess amine using a 9:1 CHCl3:MeOH with a drop of ammonium hydroxide as eluent. Total yield of this syrupy ring-opened product was 0.40 g. The product was confirmed by NMR and MS.

The product was further deprotected using catalytic amounts of sodium methoxide in 20 ml of anhydrous methanol. TLC (Thin layer chromatography) showed that after 8 hours the product was completely deacetylated. The deacetylated product was passed through a short silica-gel column (8:2 CHCl2:methanol) to yield .175 g of the final product, 3-(N,N-dibutylamino)-2-hydroxypropyl-beta-D-galactopyranoside.

EXAMPLE 2

In a 50ml, 2 neck flask is added 1 gram of 2,3-epoxypropyl beta-D galactopyranoside 2,3,4,6-0-tetraacetate (epoxide compound), 10ml of anhydrous acetonitrile, and 2-10 equivalents of a primary or secondary amine. The reaction is allowed to stir at 20-80°C and is followed by thin layer chromatography using a 9:1 CHCl3:MeOH with a drop of ammonium hydroxide as eluent. More amine can be added to ensure all the epoxide is reacted. The ring-opened product is isolated and the acetate groups are removed if necessary, with catalytic amounts of sodium methoxide in anhydrous methanol. The product is further purified by passing through a silica gel column.

With regard to Examples 1 and 2 above, while it would not necessarily be expected for the 3-(N,N-dibutylamino)-2-hydroxypropyl-beta-D-galactopyranoside of Example 1 to have optimal surfactancy (because of butyl groups), longer chain groups (eg $C_6$ - $C_{20}$, preferably $C_8$-$C_{14}$) can be prepared in the same manner or as described in Example 2 (where the primary or secondary amine added to epoxide would have, for example, $C_{10}$-$C_{14}$ groups). Preferably, in Example 2, a n-monoalkylamine (eg $NH_2C_{12}H_{25}$) would be used.

EXAMPLE 3

This example describes a study of the detergency of a glyceroglycolipid surfactant in accordance with the invention, 1-N-dodecyl-3-hydroxypropylamine-3-0-galactopyranoside (DHAG).

Its Krafft Temperature was found to be <8°C.

Detergency performance was assessed in triolein removal experiments using a Terg-O-Tometer. $^3H$ radio-labelled triolein was used to assess soil removal; subsequent to the wash, 4 x 1 ml samples of wash liquor was removed from each pot and the activity determined using a liquid scintillation counter. Percentage detergency was calculated from the relationship:

$$\% \text{ Detergency} = \frac{A_w \times 100}{A_s}$$

where $A_w$ is the total activity in the wash liquor and $A_s$ is the level of activity originally applied to the cloth.

Wash conditions are given below:

| | |
|---|---|
| Apparatus | Terg-O-Tometer UR 7227 |
| Wash time | 20 minutes |
| Agitation | 70 rpm |
| Wash liquide volume | 500 mls |
| Dosage | 1 g/l |
| Salt concentration (carbonate of metaborate) | 0.05M |
| Test cloth | Knitted polyester |
| Soil level | ca. 1.9% |

The detergency performance of DHAG,in triolein removal experiments at 40°C, with varying ratios of triethylene glycol mono-dodecyl ether ($C_{12}$ alcohol 3EO ethoxylate) ($C_{12}EO_3$) is shown in Figures 1 and 2 of the accompanying drawings, for experiments performed in carbonate and borate buffered solutions respectively.

DHAG on its own gave triolein removal of about 58% which is comparable to that obtained with $C_{12}EO_8$ or $C_{12}EO_6$ alone. Mixtures of DHAG and $C_{12}EO_3$ gave a small synergistic improvement. Similar detergency results were obtained whether borate or carbonate-buffered solutions were used.

These results indicate DHAG to be a very promising candidate for the replacement of high HLB ethoxylated surfactants as far as oily soil detergency is concerned.

## Claims

1. A detergent or cleansing composition having a surfactant system comprising a glyceroglycolipid surfactant having an amine linkage and a cosurfactant selected from fatty acid soaps, anionic non-soap surfactants, nonionic surfactants, cationic surfactants, amphoteric surfactants and zwitterionic surfactants, the glyceroglycolipid having the structure:

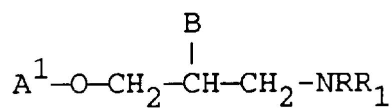

wherein $A^1$ is a saccharide, R or $R_1$ are the same or different and are hydrogen or a branched or unbranched, saturated or unsaturated, hydrocarbon radical having from 1 to 24 carbon atoms; and B is OH or $NR_2R_3$, wherein $R_2$ and $R_3$ are the same or different and are hydrogen, a branched or unbranched hydrocarbon, saturated or unsaturated radical having from 1 to 24 carbon atoms; wherein the $NRR_1$ group and B group are positionally interchangeable; and wherein at least one of R, $R_1$, $R_2$ or $R_3$ must be a hydrocarbon radical having from 1 to 24 carbon atoms.

2. A composition according to claim 1, wherein at least one of the groups R, $R_1$, $R_2$ and $R_3$ has from 6 to 22 carbon atoms.

3. A composition according to claim 3, wherein at least one of the groups R, $R_1$, $R_2$ and $R_3$ has from 10 to 14 carbon atoms.

4. A composition as claimed in claim 3, wherein one of the groups R and $R_1$ is a hydrogen atom and the other is an alkyl chain having from 10 to 14 carbon atoms.

5. A composition as claimed in claim 4, wherein one of the groups R and $R_1$ is a hydrogen atom and the other is an alkyl chain having 12 carbon atoms.

6. A composition according to any one of claims 1 to 5, wherein the saccharide $A^1$ is a monosaccharide.

7. A composition according to claim 6, wherein the monosaccharide is galactoside.

8. A composition as claimed in any one of claims 1 to 7, wherein $A^1$ is a monosaccharide, R is a hydrogen atom, $R_1$ is a hydrocarbon radical having from 6 to 18 carbon atoms, and B is OH.

9. A composition as claimed in any preceding claim, wherein the cosurfactant is an ethoxylated alcohol nonionic surfactant.

10. A composition as claimed in any preceding claim, which is a detergent or cleansing composition for fabrics or for hard surfaces.

11. A composition as claimed in claim 10, which is a liquid detergent composition comprising the following (percentages by weight):

    (1) 5-70% detergent active comprising the glyceroglycolipid surfactant having an amine linkage;
    (2) 0-50% builder;
    (3) 0-40% electrolyte;
    (4) 0.01-5% enzyme;
    (5) 0.1-15% enzyme stabiliser;

(6) 0-20% phase regulant;
(7) remainder water and minors.

**12.** A composition according to any one of claims 1 to 10, which is a powdered detergent composition comprising the following (percentages by weight):

(1) 5-40% detergent active comprising the glyceroglycolipid surfactant having an amine linkage;
(2) 0-60% builder;
(3) 0-30% electrolyte
(4) 0-30% sulphate;
(5) 0-20% bleach system;
(6) 0-4% enzyme;
(7) minors plus water to 100%.

**13.** A composition according to any one of claims 1 to 10, which is a light duty liquid detergent composition comprising the following (percentages by weight):

(a) 0.01-65% anionic surfactant;
(b) 0.1-50% glyceroglycolipid surfactant;
(c) 0-8% suds promoting agent;
(d) 0-10% hydrotrope; and
(e) minors plus water to 100%.

## Patentansprüche

**1.** Waschmittel- oder Reinigungszusammensetzung mit einem Surfactant-System, umfassend ein Glyceroglykolipid-Surfactant mit einer Amin-Bindung und ein Cosurfactant, ausgewählt aus Fettsäureseifen, anionischen Nichtseife-Surfactants, nichtionischen Surfactants, kationischen Surfactants, amphoteren Surfactants und zwitterionischen Surfactants, wobei das Glyceroglykolipid die nachfolgende Struktur aufweist:

$$A^1\text{-}O\text{-}CH_2\text{-}\overset{\overset{B}{|}}{C}H\text{-}CH_2NRR^1$$

worin $A^1$ ein Saccharid ist, R oder $R^1$ gleich oder verschieden sind und Wasserstoff oder einen verzweigten oder unverzweigten, gesättigten oder ungesättigten Kohlenwasserstoffrest mit von 1 bis 24 Kohlenstoffatomen bedeuten; und B OH oder $NR^2R^3$ ist, worin $R^2$ und $R^3$ gleich oder verschieden sind und Wasserstoff, einen verzweigten oder unverzweigten gesättigten oder ungesättigten Kohlenwasserstoffrest mit 1 bis 24 Kohlenstoffatomen bedeuten; worin die $NRR^1$-Gruppe und B in ihrer Position austauschbar sind; und worin zumindest einer der Reste R, $R^1$, $R^2$ oder $R^3$ ein Kohlenwasserstoffrest mit 1 bis 24 Kohlenstoffatomen sein muß.

**2.** Zusammensetzung nach Anspruch 1, worin zumindest eine der Gruppen R, $R^1$, $R^2$ und $R^3$ von 6 bis 22 Kohlenstoffatome aufweist.

**3.** Zusammensetzung nach Anspruch 2, worin zumindest eine der Gruppen R, $R^1$, $R^2$ und $R^3$ von 10 bis 14 Kohlenstoffatome aufweist.

**4.** Zusammensetzung nach Anspruch 3, worin eine der Gruppen R und $R^1$ ein Wasserstoffatom und die andere eine Alkylkette mit 10 bis 14 Kohlenstoffatomen bedeutet.

**5.** Zusammensetzung nach Anspruch 4, worin eine der Gruppen R und $R^1$ ein Wasserstoffatom und die andere eine Alkylkette mit 12 Kohlenstoffatomen bedeutet.

**6.** Zusammensetzung nach einem der Ansprüche 1 bis 5, worin das Saccharid $A^1$ ein Monosaccharid ist.

**7.** Zusammensetzung nach Anspruch 6, worin das Monosaccharid Galactosid ist.

**8.** Zusammensetzung nach einem der Ansprüche 1 bis 7, worin $A^1$ ein Monosaccharid, R ein Wasserstoffatom, $R^1$

ein Kohlenwasserstoffrest mit 6 bis 18 Kohlenstoffatomen und B OH ist.

**9.** Zusammensetzung nach einem der vorstehenden Ansprüche, worin das Cosurfactant ein ethoxyliertes alkoholisches nichtionisches Surfactant ist.

**10.** Zusammensetzung nach einem der vorstehenden Ansprüche, welche eine Waschmittel- oder Reinigungszusammensetzung für Gewebe oder für harte Oberflächen ist.

**11.** Zusammensetzung nach Anspruch 10, welche eine flüssige Waschmittelzusammensetzung ist, enthaltend die folgenden Bestandteile (Gewichtsprozent):

(1) 5 bis 70 % waschmittelaktive Verbindung, enthaltend das Glyceroglykolipid-Surfactant mit einer Amin-Bindung;
(2) 0 bis 50 % Builder;
(3) 0 bis 40 % Elektrolyt;
(4) 0,01 bis 5 % Enzym;
(5) 0,1 bis 15 % Enzym-Stabilisator;
(6) 0 bis 20 % Phasenreguliermittel;
(7) Rest Wasser und geringere Bestandteile.

**12.** Zusammensetzung nach einem der Ansprüche 1 bis 10, welche eine pulverisierte Waschmittelzusammensetzung ist, enthaltend die folgenden Bestandteile (Gewichtsprozent):

(1) 5 bis 40 % waschmittelaktive Verbindung, enthaltend das Glyceroglykolipid-Surfactant mit einer Amin-Bindung;
(2) 0 bis 60 % Builder;
(3) 0 bis 30 % Elektrolyt;
(4) 0 bis 30 % Sulfat;
(5) 0 bis 20 % Bleichsystem;
(6) 0 bis 4% Enzym;
(7) Geringe Bestandteile plus Wasser auf 100 %.

**13.** Zusammensetzung nach einem der Ansprüche 1 bis 10, die eine flüssige Leichtwaschmittel-Zusammensetzung ist, enthaltend die folgenden Bestandteile (Gewichtsprozent):

(a) 0,01 bis 65 % Anionisches Surfactant;
(b) 0,1 bis 50 % Glyceroglykolipid-Surfactant;
(c) 0 bis 8 % schaumförderndes Mittel;
(d) 0 bis 10 % Hydrotrop; und
(e) Geringere Bestandteile plus Wasser auf 100 %.

## Revendications

**1.** Une composition détergente ou nettoyante présentant un système tensioactif comprenant un agent tensioactif de glycéroglycolipide ayant une liaison amine et un agent co-tensioactif sélectionné parmi des savons d'acide gras, des agents tensioactifs anioniques sans savon, des agents tensioactifs non ioniques, des agents tensioactifs cationiques, des agents tensioactifs amphotères et des agents tensioactifs zwitterioniques, le glycéroglycolipide présentant la structure :

$$A^1 - O - CH_2 - \underset{}{\overset{\displaystyle B \atop \displaystyle |}{CH}} - CH_2 - NRR_1$$

où $A^1$ est un saccharide, R ou $R_1$ sont identiques ou différents et sont un hydrogène ou un radical hydrocarbure linéaire ou ramifié, saturé ou insaturé, présentant 1 à 24 atomes de carbone ; et B est OH ou $NR_2R_3$, où $R_2$ et $R_3$ sont identiques ou différents et sont un hydrogène, un hydrocarbure linéaire ou ramifié, un radical saturé ou insaturé présentant 1 à 24 atomes de carbone ; où la position du groupe $NRR_1$ et du groupe B est interchangeable ; et où

au moins un des groupes R, $R_1$, $R_2$ ou $R_3$ doit être un radical hydrocarbure présentant 1 à 24 atomes de carbone.

**2.** Une composition selon la Revendication 1, dans laquelle au moins un des groupes R, $R_1$, $R_2$ et $R_3$ présente 6 à 22 atomes de carbone.

**3.** Une composition selon la Revendication 2, dans laquelle au moins un des groupes R, $R_1$, $R_2$ et $R_3$ présente 10 à 14 atomes de carbone.

**4.** Une composition telle que revendiquée dans la Revendication 3, dans laquelle un des groupes R et $R_1$ est un atome d'hydrogène et l'autre est une chaîne alkyle présentant 10 à 14 atomes de carbone.

**5.** Une composition telle que revendiquée dans la Revendication 4, dans laquelle un des groupes R et $R_1$ est un atome d'hydrogène et l'autre est une chaîne alkyle présentant 12 atomes de carbone.

**6.** Une composition selon l'une quelconque des Revendications 1 à 5, dans laquelle le saccharide $A^1$ est un monosaccharide.

**7.** Une composition selon la Revendication 6, dans laquelle le monosaccharide est du galactoside.

**8.** Une composition telle que revendiquée dans l'une quelconque des Revendications 1 à 7, dans laquelle $A^1$ est un monosaccharide, R est un atome d'hydrogène, $R_1$ est un radical hydrocarbure présentant 6 à 18 atomes de carbone, et B est OH.

**9.** Une composition telle que revendiquée dans l'une des Revendications précédentes, dans laquelle l'agent cotensioactif est un agent tensioactif non ionique d'alcool éthoxylé.

**10.** Une composition telle que revendiquée dans l'une des Revendications précédentes, qui est une composition détergente ou nettoyante pour des tissus ou des surfaces dures.

**11.** Une composition telle que revendiquée dans la Revendication 10, qui est une composition détergente liquide comprenant les pourcentages en masse suivants :

(1) 5 à 70 % d'un détergent actifcomprenant l'agent tensioactif de glycéroglycolipide présentant une liaison amine ;
(2) 0 à 50 % d'un édificateur de détergence ;
(3) 0 à 40 % d'électrolyte ;
(4) 0,01 à 5 % d'enzyme ;
(5) 0,1 à 15 % de stabilisateur d'enzyme ;
(6) 0 à 20 % de régulateur de phase ; et
(7) le reste d'eau et d'ingrédients secondaires.

**12.** Une composition selon l'une quelconque des Revendications 1 à 10, qui est une composition détergente en poudre comprenant les pourcentages en masse suivants :

(1) 5 à 40% de détergent actifcomprenant l'agent tensioactif de glycéroglycolipide présentant une liaison amine ;
(2) 0 à 60 % d'édificateur de détergence ;
(3) 0 à 30 % d'électrolyte ;
(4) 0 à 30 % de sulfate ;
(5) 0 à 20 % d'un système de blanchiment ;
(6) 0 à 4 % d'enzyme ; et
(7) des ingrédients secondaires plus de l'eau jusqu'à 100 %.

**13.** Une composition selon l'une quelconque des Revendications 1 à 10, qui est une composition détergente liquide à bas rendement comprenant les pourcentages en masse suivants :

(1) 0,01 à 65 % d'un agent tensioactif anionique ;
(2) 0,1 à 50 % d'un agent tensioactif de glycéroglycolipide ;

(3) 0 à 8 % d'un agent favorisant la mousse ;
(4) 0 à 10 % d'hydrotrope ; et
(5) des ingrédients secondaires plus de l'eau jusqu'à 100 %.

Fig.1.

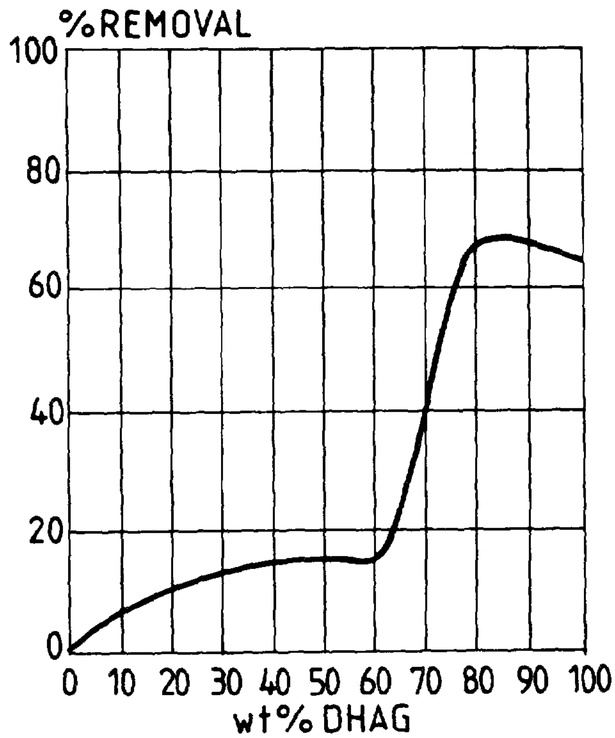
%REMOVAL
100
80
60
40
20
0
0 10 20 30 40 50 60 70 80 90 100
wt% DHAG

Fig.2.

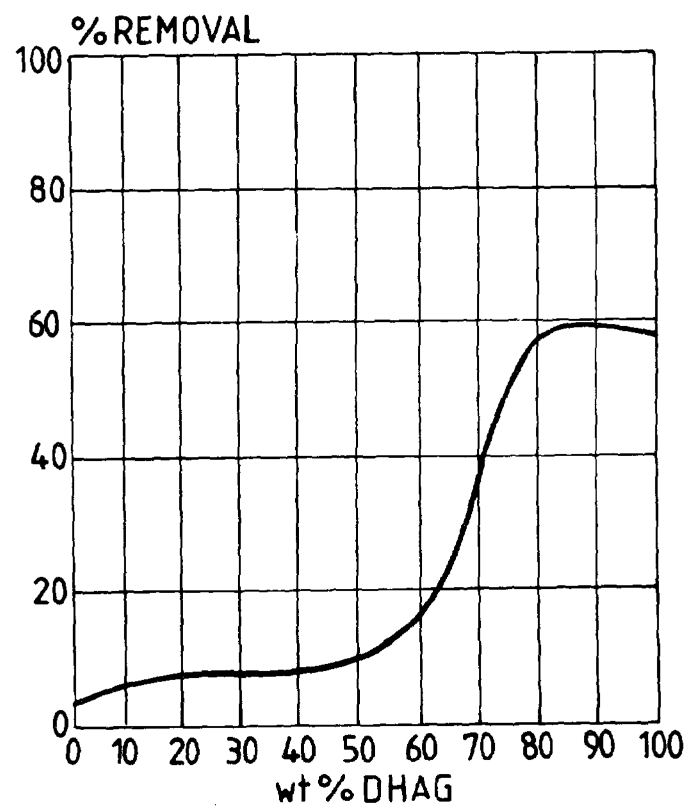
%REMOVAL
100
80
60
40
20
0
0 10 20 30 40 50 60 70 80 90 100
wt% DHAG